# EUROPÄISCHE PATENTSCHRIFT

(11) **EP 2 311 343 B1**
(45) Veröffentlichungstag und Bekanntmachung des Hinweises auf die Patenterteilung: **25.06.2014**
(21) Anmeldenummer: 10186906.3
(22) Anmeldetag: 07.10.2010
(51) Int. Cl.: A43D 1/02, A61B 5/107

(54) **Vorrichtung zur Aufzeichnung der Trittspur eines Menschen**
Device for recording the footstep of a person
Dispositif d'enregistrement de la trace de pas d'un homme

(30) Priorität: 07.10.2009 AT 15842009
(43) Veröffentlichungstag der Anmeldung: 20.04.2011
(73) Patentinhaber: Schindlegger, Christian, 3204 Kirchberg / Pielach (AT)
(72) Erfinder: Schindlegger, Christian, 3204 Kirchberg / Pielach (AT)
(74) Vertreter: Müllner, Martin

(56) Entgegenhaltungen:
- WO-A1-03/087716
- JP-A- 2001 000 207
- KR-A- 20100 110 585
- US-B1- 6 205 230

## Beschreibung

### Technisches Gebiet

Die Erfindung betrifft eine Vorrichtung zur Aufzeichnung der Trittspur eines Menschen für orthopädische Zwecke, wie beispielsweise zur Herstellung von Schuheinlagen, mit einer an der Oberseite eines Kastens vorgesehenen Glasplatte als Standfläche und einem im Kasten unterhalb der Glasplatte angeordneten, schräg stehenden Spiegel sowie mit einer Digitalkamera, deren Objektiv auf den schräg stehenden Spiegel zur Abbildung der Fußsohlen oberhalb der Glasplatte gerichtet ist.

### Stand der Technik

Somit erfolgt eine photographische Aufnahme in der Art, wie dies beispielsweise aus der DE 202006014657 U hervorgeht. Eine Vorrichtung zum Betrachten einer Fußsohle ist in der DE 2062807 A beschrieben. Eine Objektplatte als Standplatte für eine Person wird seitlich an einer Schnittkante angestrahlt und die Fußsohle von unten über einen oder mehrere Spiegel betrachtet. Die US 4534365 A zeigt einen Kasten mit einer Glasplatte als Standfläche und darunter einen schwenkbaren Spiegel, der einerseits in Richtung eines Betrachtungsschachtes und anderseits in Richtung eines Kameraschachtes geschwenkt werden kann. In der US 5128880 A ist ein Verfahren zur Ablichtung der Fußsohlen mit einem Scannerbalken beschrieben, der unterhalb der transparenten Standfläche entlangläuft und ein Bild während der Bewegung digital zusammensetzt. Ein angeschlossenes Diagnosesystem qualifiziert das Abbild nach verschiedenen Kriterien. Auch die US 4538353 A beschäftigt sich mit dem Vermessen eines Fußes, um einen Schuh in der passenden Größe auswählen zu können. In einem Ausschnitt eines Kastens wird ein Fuß auf eine transparente Platte gestellt und beleuchtet. Darunter befinden sich Spiegel, die das Abbild zu einer Kamera werfen. Das digitale Foto wird in einem Rechner analysiert und einer Schuhgröße zugeordnet.

Die Merkmale des Oberbegriffs des Anspruchs 1 sind aus WO 03/087716 A bekannt.

Alle vorgenannten Anordnungen sind groß und schwer und für den mobilen Gebrauch kaum geeignet.

### Kurzbeschreibung der Erfindung

Die Erfindung zielt darauf ab, eine kompakte Vorrichtung zu schaffen, die sehr leicht und problemlos etwa in Spitäler, in Privatwohnungen oder in Schulen transportierbar ist. Eine geringe Bauhöhe ist anzustreben, damit eine Person mit bloßen Füßen ohne Gleichgewichtsprobleme und völlig entspannt auf der flachen Vorrichtung schwankungsfrei und fest stehen kann.

Dieses Ziel wird dadurch erreicht, dass der schräg stehende Spiegel und die Kamera auf einem unterhalb der Glasplatte horizontal und quer zur Standrichtung auf der Glasplatte verfahrbaren Schlitten vorgesehen sind und dass der Schlitten über einen Antrieb verfügt, der in zwei Positionen des Schlittens zur Abbildung der einen und sodann der anderen Fußsohle abschaltbar ist. Durch ein Querverschieben eines bloß auf jeweils nur eine Fußsohle gerichteten Spiegels kann ein kleinerer Spiegel von geringer Höhe eingesetzt werden, wobei die Ablichtung der beiden Fußsohlen nacheinander erfolgt.

Dabei ist es zweckmäßig, wenn der Spiegel in einem spitzen Winkel zur Bewegungsebene des Schlittens angeordnet ist und wenn die Kamera am Schlitten nahe dem oberen Kastendeckel angeordnet ist. Dadurch nimmt der in einem flachen Winkel von z.B. 30° stehende Spiegel nur wenig Bauhöhe im Inneren des Kastens in Anspruch, sodass der Kasten als solcher sehr flach gehalten werden kann.

Eine besondere Ausführungsform der Erfindung ist dadurch gekennzeichnet, dass eine Steuerung vorgesehen ist, die als Zyklus ein Foto in der ersten Schlittenposition auslöst, den Schlittenantrieb zum Verfahren des Schlittens in die zweite Position einschaltet, nach Erreichen der zweiten Position ein zweites Foto auslöst und dann den Schlitten in die erste Position als Grundstellung zurückführt, wobei die Fotos über einen Anschluss am Kasten auf einem Bildschirm darstellbar und über einen Drucker ausdruckbar sind.

Nach Betreten der Standfläche auf einem etwa nur 15 cm hohen Kasten mit einer Grundfläche von 30 x 50 cm oder 35 x 65 cm kann der Zyklus mittels einer Fernsteuerung ausgelöst werden.

### Kurze Beschreibung der Zeichnungsfiguren

Ein Ausführungsbeispiel des Erfindungsgegenstandes ist in den Zeichnungen dargestellt.

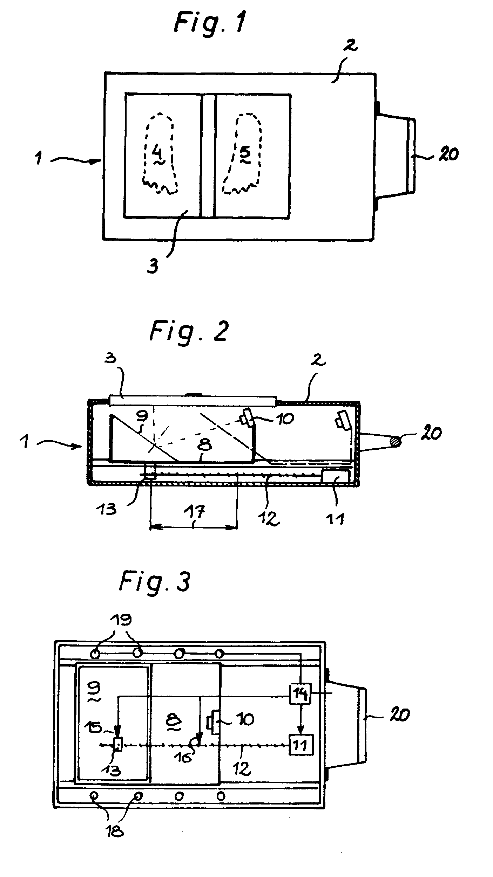

Fig. 1 zeigt eine Vorrichtung gemäß der Erfindung von oben, Fig. 2 die Vorrichtung im Querschnitt und Fig. 3 eine Ansicht bei abgenommenem Deckel.

### Beschreibung der Ausführungsarten

Eine erfindungsgemäße Vorrichtung umfasst einen flachen, quaderförmigen Kasten 1 mit einem Deckel 2, in welchem ein durch eine Glasplatte 3 geschlossenes Fenster vorgesehen ist. Die Glasplatte 3 einschließlich des Kastens 1 ist so stabil, dass sie das Gewicht eines Menschen problemlos tragen kann. Die Glasplatte 3 bildet eine Standfläche für beide (bloßen) Füße zum Zwecke der Aufzeichnung der Trittspur des Menschen.

Im Inneren des Kastens 1, dessen Abmessungen bei einem Ausführungsbeispiel etwa 30 cm (Breite) x 50 cm (Länge) x 15 cm (Höhe) betragen, laufen entlang der Längsseite Schienen 6, 7, in welchen ein Schlitten 8 quer zur Standrichtung des Menschen auf der Glasplatte 3 verfahrbar gelagert ist. Der Schlitten 8 trägt an einem Ende einen schräg stehenden Spiegel 9 und am anderen Ende in erhöhter Position eine Kamera 10. Ein Antrieb, hier in Form einer durch einen Elektromotor 11 angetriebenen Gewindespindel 12, auf welcher eine mit dem Schlitten 8 fest verbundene Mutter 13 läuft, verschiebt den Schlitten 8 derart, dass in einer Schlittenposition (mit vollen Linien in Fig. 2 dargestellt) der Spiegel 9 unterhalb der rechten Fußsohle 4 positioniert ist. Die relativ zum Spiegel 9 starr auf dem Schlitten 8 erhöht positionierte Kamera kann ein Foto der rechten Fußsohle 4 aufzeichnen. Auch ein Zahnriemenantrieb ist zur Positionierung des Schlittens 8 bestens geeignet.

Mittels des Antriebes wird sodann der Schlitten 8 quer zur Standrichtung so weit nach rechts verfahren, bis sich der Spiegel 9 unter der linken Fußsohle 5 befindet. Diese Position ist in Fig. 2 mit strichlierten Linien angedeutet. Es wird nun ein zweites Foto der linken Fußsohle 5 aufgenommen.

In Fig. 3 ist eine dem Motor 11 zugeordnete Steuerung 14 dargestellt, die mit Endschaltern im Verschiebungsbereich des Schlittens 8 verbunden ist. Diese Endschalter sind durch Pfeile 15, 16 symbolisiert. Es können mechanische Kontakte oder Reed-Kontakte sein, die von einem am Schlitten 8 angeordneten Magneten ausgelöst werden und so die beiden Positionen des Schlittens 8 gemäß Fig. 2 vorgeben. Der Verschiebungsweg ist in Fig. 2 durch den Pfeil 17 gekennzeichnet. Die Steuerung 14 löst ferner auch in jeder der beiden Positionen die Kamera 10 aus.

Im Ausführungsbeispiel wird eine durch Lampen 18 und 19 dargestellte Beleuchtung im Bodenbereich seitlich neben dem Schlitten 8 schon bei Verbindung der Vorrichtung mit dem Stromnetz eingeschaltet. Dies hat bei Glühlampen den Vorteil, dass die Glasplatte 3 bereits vor dem Betreten mit bloßen Füßen erwärmt wird. Ferner leuchtet die Glasplatte 3 permanent und nicht etwa erst im Augenblick der Fotoaufnahme, was zu einer Irritation des Menschen führt. Diese (unnötige) Schrecksekunde hätte ein Zucken und damit eine Unschärfe im Foto zur Folge. Natürlich können auch LED's oder Leuchtstoffröhren zur Beleuchtung verwendet werden.

Die Zyklusauslösung über die Steuerung 14 kann mittels eines Funksenders erfolgen, sobald ein Mensch die erforderliche Standposition auf der Glasplatte 3 eingenommen hat. Es wird ein Foto der rechten Fußsohle 4 ausgelöst, der Schlitten 8 unter die linke Fußsohle 5 verschoben, dann ein weiteres Foto ausgelöst und schließlich der Schlitten 8 wieder in die Grundstellung (unter die rechte Fußsohle 4) gebracht. Damit ist der Zyklus beendet. Ein Funkbefehl startet einen neuen Zyklus. Natürlich kann auch ein Schalter oder Taster auf einem Handauslöser mit einer steckbaren Kabelverbindung zum Kasten 1 und zur Steuerung 14 vorgesehen sein. Ein kleines Display kann auf dem Handauslöser bzw. mit Funkverbindung zur Kamera die Untersicht der jeweiligen Fußsohle zeigen.

Die Kamera 10 ist mit einem Anschluss ausgestattet, der in der Nähe eines Transportgriffes 20 herausgeführt ist und die sofortige Betrachtung der Fotos sowie das Ausdrucken gestattet.

Die Glasplatte 3 kann natürlich auch aus einem anderen transparenten Material (z.B. Acrylglas an Stelle von Mineralglas) bestehen.

Einer besonderen Erwähnung bedarf die Schrägstellung des Spiegels 9 in einem spitzen Winkel zur Verschiebungsebene des Schlittens 8. Um eine geringe Bauhöhe des Kastens 1 zu erzielen, ist der Spiegel nicht etwa in einem Winkel von 45° zur Horizontalen angeordnet, sondern in einem wesentlich flacheren Winkel - wie dargestellt. Natürlich ist dann aus Gründen der Reflexion die Kamera 10 am Schlitten 8 knapp unterhalb des Deckels 2 (bzw. der Glasplatte 3) zu positionieren.

## Patentansprüche

1. Vorrichtung zur Aufzeichnung der Trittspur eines Menschen für orthopädische Zwecke, wie beispielsweise zur Herstellung von Schuheinlagen, mit einer an der Oberseite eines Kastens (1) vorgesehenen Glasplatte (3) als Standfläche und einem im Kasten (1) unterhalb der Glasplatte (3) angeordneten, schräg stehenden Spiegel (9) sowie mit einer Digitalkamera (10), deren Objektiv auf den schräg stehenden Spiegel (9) zur Abbildung der Fußsohlen (4, 5) oberhalb der Glasplatte (3) gerichtet ist, wobei der schräg stehende Spiegel (9) und die Kamera (10) auf einem unterhalb der Glasplatte (3) horizontal und quer zur Standrichtung auf der Glasplatte (3) verfahrbaren Schlitten (8) vorgesehen sind, **dadurch gekennzeichnet, dass** der Schlitten (8) über einen Antrieb verfügt, der in zwei Positionen des Schlittens (8) zur Abbildung der einen und sodann der anderen Fußsohle (4, 5) abschaltbar ist.

2. Vorrichtung nach Anspruch 1, **dadurch gekennzeichnet, dass** der Spiegel (9) in einem spitzen Winkel zur Bewegungsebene des Schlittens (8) angeordnet ist und dass die Kamera (10) am Schlitten (8) nahe dem oberen Kastendeckel (2) vorgesehen ist.

3. Vorrichtung nach Anspruch 1, **dadurch gekennzeichnet, dass** eine Steuerung (14) vorgesehen ist, die als Zyklus ein Foto in der ersten Schlittenposition auslöst, den Schlittenantrieb zum Verfahren des Schlittens (8) in die zweite Position einschaltet, nach Erreichen der zweiten Position ein zweites Foto auslöst und dann den Schlitten (8) in die erste Position als Grundstellung zurückführt, wobei die Fotos über einen Anschluss am Kasten (1) auf einem Bildschirm darstellbar und über einen Drucker ausdruckbar sind.

4. Vorrichtung nach Anspruch 3, **dadurch gekennzeichnet, dass** der Zyklus mittels einer Fernsteuerung auslösbar ist.

## Claims

1. A device for recording the footprint of a person for orthopaedic purposes, such as, for example, for the production of shoe inserts, having a glass plate (3) as a standing surface provided on the upper side of a box (1), and an obliquely standing mirror (9) arranged in the box (1) underneath the glass plate (3), and also having a digital camera (10), whose lens is directed at the obliquely standing mirror (9) for imaging the soles (4, 5) of the feet above the glass plate (3), wherein the obliquely standing mirror (9) and the camera (10) are provided on a slide (8) which can be moved underneath the glass plate (3) horizontally and transversely to the standing direction on the glass plate (3), **characterised in that** the slide (8) is equipped with a drive which can be switched off in two positions of the slide (8) in order to image the one and then the other sole (4, 5) of the feet.

2. A device according to claim 1, **characterised in that** the mirror (9) is arranged at an acute angle to the plane of movement of the slide (8), and **in that** the camera (10) is provided on the slide (8) close to the upper box lid (2).

3. A device according to claim 1, **characterised in that** a control (14) is provided which, as a cycle, triggers a photo in the first slide position, switches on the slide-drive to move the slide (8) into the second position, after the second position is reached triggers a second photo and then guides the slide (8) back into the first position as the basic position, wherein the photos can be presented on a screen by way of a connection on the box (1) and can be printed out by way of a printer.

4. A device according to claim 3, **characterised in that** the cycle can be triggered by means of a remote control.

## Revendications

1. Dispositif qui permet d'enregistrer l'empreinte d'un pied humain à des fins d'orthopédie, par exemple pour fabriquer des semelles orthopédiques, et qui est pourvu d'une plaque en verre (3) laquelle est disposée sur la face supérieure d'un caisson (1) et laquelle sert de support pour une personne en position debout, et d'un miroir (9) incliné lequel est disposé dans le caisson (1) en-dessous de la plaque en verre (3), ainsi que d'une caméra numérique (10) dont l'objectif vise le miroir (9) incliné, permettant ainsi de réaliser une prise de vue des plantes de pied (4, 5) se trouvant en-dessus de la plaque en verre (3), le miroir (9) incliné et la caméra (10) étant disposés sur un chariot (8) pouvant se déplacer horizontalement, en-dessous de la plaque en verre (3), dans une direction transversale à l'orientation de la personne se tenant debout sur la plaque en verre (3), **caractérisé en ce que** le chariot (8) est pourvu d'un organe d'entraînement lequel peut être désactivé à deux positions du chariot (8) pour ainsi réaliser une prise de vue de l'une des plantes de pied (4, 5) puis de l'autre.

2. Dispositif selon la revendication 1, **caractérisé en ce que** le miroir (9) et le plan dans lequel se déplace le chariot (8) forment un angle aigu, et que la caméra (10) est disposée sur le chariot (8) à proximité du couvercle supérieur (2) du caisson.

3. Dispositif selon la revendication 1, **caractérisé en ce qu'**il est pourvu d'un organe de commande (14) permettant de réaliser un cycle selon lequel une prise de vue est déclenchée dans la première position du chariot, l'organe d'entraînement du chariot est activé pour déplacer le chariot (8) vers la deuxième position, une deuxième prise de vue est déclenchée une fois la deuxième position atteinte, pour ensuite ramener le chariot (8) dans la première position qu'il adopte par défaut, une connexion sur le caisson (1) permettant d'afficher les prises de vue sur un écran et de les imprimer au moyen d'une imprimante.

4. Dispositif selon la revendication 3, **caractérisé en ce que** ledit cycle peut être déclenché au moyen d'une télécommande.
